(19) Europäisches Patentamt
European Patent Office
Office européen des brevets

(11) EP 1 515 750 B1

(12) EUROPEAN PATENT SPECIFICATION

(45) Date of publication and mention of the grant of the patent:
**11.08.2010 Bulletin 2010/32**

(21) Application number: **03757040.5**

(22) Date of filing: **06.06.2003**

(51) Int Cl.:
***A61K 45/06*** *(2006.01)* ***A61K 31/426*** *(2006.01)*
***A61K 31/427*** *(2006.01)* ***A61P 35/00*** *(2006.01)*

(86) International application number:
**PCT/EP2003/005988**

(87) International publication number:
**WO 2003/103712 (18.12.2003 Gazette 2003/51)**

(54) **COMBINATIONS COMPRISING EPOTHILONES AND PHARMACEUTICAL USES THEREOF**

EPOTHILONE ENTHALTENDE KOMBINATIONEN UND DEREN PHARMAZEUTISCHE VERWENDUNGEN

COMBINAISONS COMPRENANT DES EPOTHILONES, ET UTILISATIONS PHARMACEUTIQUES DE CELLES-CI

(84) Designated Contracting States:
**AT BE BG CH CY CZ DE DK EE ES FI FR GB GR HU IE IT LI LU MC NL PT RO SE SI SK TR**

(30) Priority: **10.06.2002 US 387025 P**
**27.08.2002 US 406238 P**
**27.08.2002 US 406239 P**
**08.01.2003 US 438677 P**
**08.01.2003 US 438676 P**

(43) Date of publication of application:
**23.03.2005 Bulletin 2005/12**

(60) Divisional application:
**09178302.7 / 2 179 745**

(73) Proprietors:
- **Novartis AG**
**4056 Basel (CH)**
Designated Contracting States:
**BE BG CH CY CZ DE DK EE ES FI FR GB GR HU IE IT LI LU MC NL PT RO SE SI SK TR**
- **Novartis Pharma GmbH**
**1230 Wien (AT)**
Designated Contracting States:
**AT**

(72) Inventors:
- **HOHNEKER, John, Arthur**
**Morristown, NJ 07960 (US)**
- **MILLER, Julie, Ann**
**Whippany, NJ 07981 (US)**
- **ROTHERMEL, John, David**
**Randolph, NJ 07869 (US)**
- **WARTMANN, Markus**
**CH-4125 Riehen (CH)**

(74) Representative: **de Weerd, Petrus G.W. et al**
**Novartis International AG**
**Corporate Intellectual Property**
**4002 Basel (CH)**

(56) References cited:
**WO-A-00/47584** **WO-A-01/64650**
**WO-A-01/72721** **WO-A-01/81341**
**WO-A-01/81342** **WO-A-99/01124**
**WO-A-02/072085** **US-A1- 2002 058 286**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 515 750 B1

## Description

The invention relates to a pharmaceutical combination which comprises (a) at least one antineoplastic agent selected from the topoisomerase I inhibitors, or topoisomerase II inhibitors as defined in claim 1, and (b) epothilone B and optionally at least one pharmaceutically acceptable carrier for simultaneous, separate or sequential use, in particular, for the treatment of a proliferative disease, especially a solid tumor disease; a pharmaceutical composition comprising such a combination; the use of such a combination for the preparation of a medicament for the treatment of a proliferative disease; a commercial package or product comprising such a combination as a combined preparation for simultaneous, separate or sequential use.

Despite the widespread use of Taxol® and Taxotere® in the treatment of many different tumor types, the impact of taxanes on patient survival has been modest, and the overwhelming majority of metastatic solid tumors remain incurable. Taxane treatment is associated with a number of significant side-effects, and the effectiveness of taxanes can be severely limited by the rapid development of drug resistance mechanisms. In view of these limitations as well as the side-effects commonly observed with standard combination therapies, there is a clear need for the identification of novel cytotoxic anti-cancer agents exhibiting an improved overall profile including spectrum of anti-tumor activity, efficacy against multi-drug resistant tumors, safety and tolerability.

The microtubule-stabilizing effect of the epothilones was first described by Bollag et al., Cancer Research 55, 1995, 2325-33. A suitable treatment schedule for the treatment of different types of tumors, especially tumors which are refractory to the treatment by other chemotherapeutics, in particular TAXOL™, using an epothilone, in particular epothilone A or B, is described in WO 99/43320.

Surprisingly, it has now been found that the anti-proliferative effect of a combination as defined herein is greater than the effect that can be achieved with either type of combination partner alone, i.e. greater than the effect of a monotherapy using only one of the combination partners (a) and (b) as defined herein.

Hence, the present invention pertains to a combination, such as a combined preparation or a pharmaceutical composition, which comprises (a) topoisomerase I inhibitors, or topoisomerase II inhibitors selected from epirubicine, doxorubicin, topotecan or 9-nitrocamptothecin and (b) epothilone and (b) an epothilone derivative epithilone B falling under the formula I

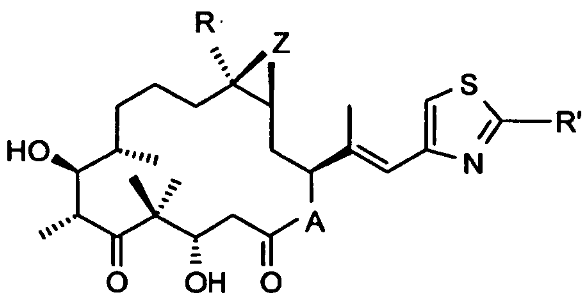

(I)

wherein A represents O, R is methyl, R' is methyl, , and Z is O, in which the active ingredients (a) and (b) are present in each case in free form or in the form of a pharmaceutically acceptable salt and optionally at least one pharmaceutically acceptable carrier; for simultaneous, separate or sequential use.

Furthermore, the present invention relates to a uses of said combination for the preparation of a medicament for of treating a warm-blooded animal having a proliferative disease comprising administering to the animal a combination according to claim 1 in a quantity which is jointly therapeutically effective against a proliferative disease and in which the compounds can also be present in the form of their pharmaceutically acceptable salts.

Unless stated otherwise, in the present disclosure organic radicals and compounds designated "lower" contain not more than 7, preferably not more than 4, carbon atoms.

A compound of formula I wherein A represents O, R is hydrogen and Z is O is known as epothilone A; a compound of formula I wherein A represents O, R is methyl and Z is O is known as epothilone B; a compound of formula I wherein A represents O, R is hydrogen and Z is a bond is known as epothilone C; a compound of formula I wherein A represents O, R is methyl and Z is a bond is known as epothilone D.

The compounds used as combination partners (a), and (b) disclosed herein can be prepared and administered as described in the cited documents, respectively, if not mentioned otherwise.

Epothilone derivatives of formula I wherein A represents O or $NR_N$, wherein $R_N$ is hydrogen or lower alkyl, R is hydrogen or lower alkyl and Z is O or a bond, and methods for the preparation of such epothilone derivatives are in

particular generically and specifically disclosed in the patents and patent applications WO 93/10121, US 6,194,181, WO 98/25929, WO 98/08849, WO 99/43653, WO 98/22461 and WO 00/31247 in each case in particular in the compound claims and the final products of the working examples, the subject-matter of the final products, the pharmaceutical preparations and the claims is hereby incorporated into the present application by reference to this publications. Comprised are likewise the corresponding stereoisomers as well as the corresponding crystal modifications, e.g. solvates and polymorphs, which are disclosed therein.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I wherein $R_N$ is lower alkyl can be prepared by methods known in the art such as a reductive alkylation reaction starting from the epothilone derivative wherein $R_N$ is hydrogen.

Epothilone derivatives of formula I wherein A represents O or $NR_N$, wherein $R_N$ is hydrogen or lower alkyl, R is hydrogen or lower alkyl, R' is methoxy, ethoxy, amino, methylamino, dimethylamino, aminomethyl or methylthio, and Z is O or a bond, and methods for the preparation and administration of such epothilone derivatives are in particular generically and specifically disclosed in the patent application WO99/67252. Comprised are likewise the corresponding stereoisomers as well as the corresponding crystal modifications, e.g. solvates and polymorphs, which are disclosed therein.

A compound of formula I, wherein A represents O, R is methyl, R' is aminomethyl and Z is O can be prepared as described in Examples 13 to 16 of WO01/72721 starting with a compound of formula I, wherein A represents O, R and R' are both methyl and Z is O.

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b) or such that the effect of a combination partner (b) is potentiated due to the presence of a combination partner (a). The ratio of the total amounts of the combination partner (a) to the combination partner (b) to be administered in the combined preparation can be varied, e.g. in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, potentiation, i.e. a combined therapeutical effect in a non-effective dosage of one or both of the combination partners (a) and (b), and very preferably a strong synergism of the combination partners (a) and (b).

The term "solid tumor disease" as used herein comprises, but is not restricted to glioma, thyroid cancer, breast cancer, especially breast tumors positive for the overexpression of HER2/neu, ovarian cancer, cancer of the colon and generally the GI tract, cervix cancer, lung cancer, in particular small-cell lung cancer, and non-small-cell lung cancer, head and neck cancer, bladder cancer, cancer of the prostate, hepatoma or Kaposi's sarcoma. Depending on the tumor type and the particular combination used, a decrease of the tumor volume can be obtained. The combinations disclosed herein are also suited to prevent the metastatic spread of tumors and the growth or development of micrometastases.

The term "proliferative disease" comprises a solid tumor disease, a liquid tumor, e.g. leukemia, and psoriasis.

The term "solid tumor disease" especially means breast cancer, cancer of the colon and generally the GI tract including gastric cancer, hepatoma; lung cancer, in particular small-cell lung cancer and non-small-cell lung cancer, renal cancer, mesothelioma, glioma, squamous cell carcinoma of the skin, head and neck cancer, genitourinary cancer, e.g. cervical, uterine, ovarian, testicles, prostate or bladder cancer; Hodgkin's disease, carcinoid syndrome or Kaposi's sarcoma. In a preferred embodiment of the invention, the solid tumor disease to be treated is selected from breast cancer, colorectal cancer, ovarian cancer, renal cancer, lung cancer, especially non-small-cell lung cancer, and glioma. Depending on the tumor type and the particular combination used a decrease of the tumor volume can be obtained. The combinations disclosed herein are also suitable to prevent the metastatic spread of tumors and the growth or development of micrometastases.

The term "topoisomerase I inhibitors" as used herein indudes, topotecan, 9-nitrocamptothecin. Topotecan can be administered, e.g., in the form as it is marketed, e.g. under the trademark HYCAMTIN™.

The term "topoisomerase II inhibitors" as used herein includes the antracyclines doxorubicin (including liposomal formulation, e.g. CAELYX™), epirubicin. Doxorubicin can be administered, e.g., in the form as it is marketed, e.g. under the trademark ADRIBLASTIN™. Epirubicin can be administered, e.g., in the form as it is marketed, e.g. under the trademark FARMORUBICIN™.

The structure of the active ingredients identified by code nos., generic or trade names may be taken from the

actual edition of the standard compendium "The Merck Index" or from databases, e.g. Patents International (e.g. IMS World Publications).

It will be understood that references to the combination partners (a) and (b) are meant to also include the pharmaceutically acceptable salts. If these combination partners (a) and (b) have, for example, at least one basic center, they can form acid addition salts. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization.

The compounds used as combination partners (a) and (b) disclosed herein can be prepared and administered as described in the cited documents, respectively. Epothilone derivatives of formula I, especially epothilone B, can be administered as part of pharmaceutical compositions which are disclosed in WO 99/39694.

The invention thus relates to a combination which comprises (a) at least one antineoplastic agent selected from the topoisomerase I inhibitors or topoisomerase II inhibitors, selected from epirubicine, doxorubicin, topotecan or 9-nitrocamptothecin and (b) epothilone B of formula I

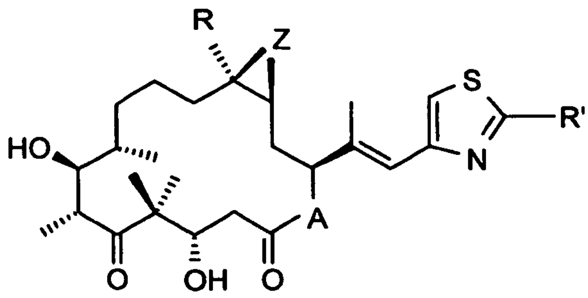

(I)

wherein A represents O, R is methyl, R' is methyl, and Z is O,

in which the active ingredients (a) and (b) are present in each case in free form or in the form of a pharmaceutically acceptable salt and optionally at least one pharmaceutically acceptable carrier; for simultaneous, separate or sequential use.

Another preferred embodiment provides combination which comprises (a) at least one antineoplastic agent selected from the topoisomerase I inhibitors, or topoisomerase II inhibitors epirubicine, doxorubicin, topotecan or 9-nitrocamptothecin and (b) epothilone B of formula I wherein A represents O, R is methyl, R' is methyl, and Z is O., in which the active ingredients are present in each case in free form or in the form of a pharmaceutically acceptable salt and optionally at least one pharmaceutically acceptable carrier; for simultaneous, separate or sequential use.

An even more preferred embodiment is a combination which comprises (a) at least one antineoplastic agent selected from the topoisomerase I inhibitors, or topoisomerase II inhibitors, epirubicine, doxorubicin, topotecan or 9-nitrocamptothecin and (b) epothilone B, in which the active ingredients are present in each case in free form or in the form of a pharmaceutically acceptable salt and optionally at least one pharmaceutically acceptable carrier; for simultaneous, separate or sequential use.

The nature of proliferative diseases like solid tumor diseases is multifactorial. Under certain circumstances, drugs with different mechanisms of action may be combined. However, just considering any combination of drugs having different mode of action does not necessarily lead to combinations with advantageous effects.

The subject-matter of the final products, the pharmaceutical preparations and the claims of the patent rights cited herein-above is hereby incorporated into the present application by reference. Comprised are likewise the corresponding stereoisomers as well as the corresponding crystal modifications, e.g. solvates and polymorphs, which are disclosed therein. The compounds used as active ingredients in the combinations disclosed herein can be prepared and administered as described in the cited documents, respectively, if not otherwise mentioned herein.

All the more surprising is the experimental finding that *in vivo* the administration of a COMBINATION OF THE INVENTION, results not only in a beneficial effect, especially a synergistic therapeutic effect, e.g. with regard to slowing down, arresting or reversing the neoplasm formation or a longer duration of tumor response, but also in further surprising beneficial effects, e.g. less side-effects, an improved quality of life and a decreased mortality and morbidity, compared to a equiefficacious monotherapy applying only one of the pharmaceutically active ingredients used in the COMBINATION OF THE INVENTION, in particular in the treatment of a tumor that is refractory to other chemotherapeutics known as anti-cancer agents.

A further benefit is that lower doses of the active ingredients of the COMBINATION OF THE INVENTION can be used, for example, that the dosages of at least one combination partner need not only often be smaller, but are also

applied less frequently, in order to diminish the incidence of side-effects. This is in accordance with the desires and requirements of the patients to be treated.

It can be shown by established test models and in particular those test models described herein that a COMBINATION OF THE INVENTION results in a more effective delay of progression or treatment of a proliferative disease compared to the effects observed with the single combination partners. The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

Suitable clinical studies are in particular open label non-randomized, dose escalation studies or placebo-controlled, double-blind studies in patients with advanced solid tumors. Such studies prove in particular the synergism of the active ingredients of the COMBINATIONS OF THE INVENTION. The beneficial effects on proliferative diseases can be determined directly through the results of these studies or by changes in the study design which are known as such to a person skilled in the art. Such studies are, in particular, suitable to compare the effects of a monotherapy using the active ingredients and a COMBINATION OF THE INVENTION.

Preferably, the combination partner (a) is administered with a fixed dose and the dose of the combination partner (b) is escalated until the Maximum Tolerated Dosage (MTD) is reached. The efficacy of the treatment can be determined in such studies, e.g., after 18 or 24 weeks by radiologic evaluation of the tumors every 6 weeks.

Alternatively, a placebo-controlled, double blind study can be used in order to prove the benefits of the COMBINATION OF THE INVENTION mentioned herein.

In a dose escalation study, the dose of the epothilone derivative of formula I, e.g. epothilone B, is escalated. In a preferred embodiment of the study, the epothilone derivative of formula I is administered once weekly i.v. for three weeks, followed by one week off starting, e.g., with 0.5 $mg/m^2$ epothilone B and escalating the dose to 1.0 $mg/m^2$, 1.5 $mg/m^2$, 2.0 $mg/m^2$ and 2.5 $mg/m^2$ until the Maximum Tolerated Dosage is reached. Each four week interval will be considered one cycle. The efficacy of the treatment can be determined in these studies, e.g., after 18 or 24 weeks by radiological evaluation of the tumors every 6 weeks.

The response criteria relating to the evaluation of target lesions to be applied in a clinical study, e.g. a breast cancer study, as described above are defined as follows:

Complete Response (CR): disappearance of all target lesions.

Partial Response (PR): at least a 30% decrease in the sum of the longest diameter (LD) of target lesions, taking as reference the baseline sum LD.

Stable Disease (SD): Neither sufficient shrinkage to qualify for PR nor sufficient increase to qualify for PD, taking as reference the smallest sum LD since the treatment started.

Progressive Disease (PD): at least a 20% increase in the sum of the LD of target lesions, taking as reference the smallest sum LD recorded since the treatment started or the appearance of one or more new lesions.

It is one objective of the present invention to provide the use of a combination of claim 1 for the manufacture of a medicament for the treatment of a proliferative disease applying a COMBINATION OF THE INVENTION having a higher rate in CR, PR or SD compared to monotherapy applying only one combination partner.

The COMBINATION OF THE INVENTION can also be applied in combination with surgical intervention, mild prolonged whole body hyperthermia and/or irradiation therapy.

One embodiment of the invention relates to the use of a COMBINATION OF THE INVENTION for the treatment of or for the preparation of a medicament for the treatment of a solid tumor disease.

It is a further objective of this invention to provide a pharmaceutical composition comprising a quantity, which is jointly therapeutically effective against a proliferative disease comprising the COMBINATION OF THE INVENTION. In this composition, the combination partners (a) or (b), and (c) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

The novel pharmaceutical composition contains, for example, from about 10 % to about 100%, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be

appreciated that the unit content of a combination partner contained in an individual dose of each dosage form need not in itself constitute an effective amount since the necessary effective amount can be reached by administration of a plurality of dosage units.

In preparing the compositions for oral dosage form, any of the usual pharmaceutical media may be employed, such as, for example, waster, glycols, oils, alcohols, flavoring agents, preservatives, coloring agents; or carriers such as starches, sugars, microcristalline cellulose, diluents, granulating agents, lubricants, binders, disintegrating agents and the like in the case of oral solid preparations such as, for example, powders, capsules and tablets, with the solid oral preparations being preferred over the liquid preparations.

In particular, a therapeutically effective amount of each of the combination partner of the COMBINATION OF THE INVENTION may be administered simultaneously or sequentially and in any order, and the components may be administered separately or as a fixed combination. For example, the method of treatment of a proliferative disease according to the invention may comprise (i) administration of the first combination partner in free or pharmaceutically acceptable salt form and (ii) adminstration of the second combination partner in free or pharmaceutically acceptable salt form, simultaneously or sequentially in any order, in jointly therapeutically effective amounts, preferably in synergistically effective amounts, e.g. in daily or weekly dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen for the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise.

If the the warm-blooded animal is a human, the dosage of a compound of formula I is preferably in the range of about 0.25 to 75, preferably 0.5 to 50, e.g. 2.5, mg/m$^2$ once weekly for two to four, e.g. three, weeks, followed by 6 to 8 days off in the case of an adult patient. In one embodiment of the invention, epothilone B is administered in accordance with the treatment schedule described in US 6,302,838 which disclosure is enclosed herein by reference.

Epothilone B is preferably administered in a dose which is calculated according to the formula (III)

$$\text{single dose (mg/m2)} = (0.1 \text{ to } y) \times N \qquad \text{(III)}$$

wherein N is the number of weeks between treatments and y is 6, wherein epothilone B is administered in more than one treatment cycle after an interval of one week to six weeks after the preceding treatment.

In one preferred embodiment of the invention, epothilone B is administered weekly in a dose that is between about 0.1 to 6 mg/m$^2$, preferably between 0.1 and 3 mg/m$^2$, e.g. 2.5 or 3.0 mg/m$^2$, for three weeks after an interval of one to six weeks, especially an interval of one week, after the preceding treatment. In another embodiment of the invention said epothilone B is preferably administered to a human every 18 to 24 days in a dose that is between about 0.3 and 12 mg/m$^2$.

Vinblastine may be administered to a human in a dosage range varying from about 1.5 to 10 mg/m$^2$day.

Doxorubicin may be administered to a human in a dosage range varying from about 10 to 100 mg/m$^2$day, e.g. 25 or 50 mg/m$^2$day.

Epirubicin may be administered to a human in a dosage range varying from about 10 to 200 mg/m$^2$day.

Topotecan may be administered to a human in a dosage range varying from about 1 to 5 mg/m$^2$day.

Furthermore, the present invention pertains to the use of a COMBINATION OF THE INVENTION for the treatment of a proliferative disease and for the preparation of a medicament for the treatment of a proliferative disease. In the compound of formula I preferably A represents O, R is methyl, R' is methyl, Z is O.

One further aspect of the present invention is the use of (a) at least one antineoplastic agent selected from the topoisomerase I inhibitors, or topoisomerase II inhibitors, epirubicine, doxorubicin, topotecan or 9-nitrocamptothecin in combination with (b) epothilone B of formula I

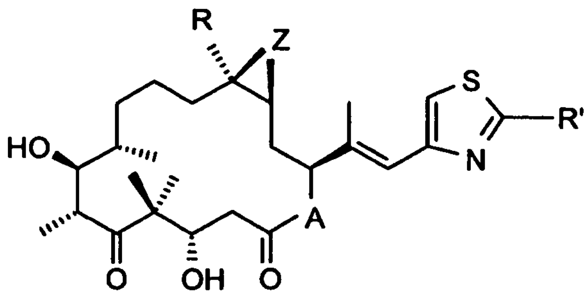

(I)

wherein A represents O, R is methyl, R' is methyl, and Z is O, for the preparation of a medicament for the treatment of a proliferative disease.

The COMBINATION OF THE INVENTION can be a combined preparation or a pharmaceutical composition.

Moreover, the present invention relates to uses in treating a warm-blooded animal having a proliferative disease comprising administering to the animal a COMBINATION OF THE INVENTION in a quantity which is jointly therapeutically effective against a proliferative disease and in which the combination partners can also be present in the form of their pharmaceutically acceptable salts.

Furthermore, the present invention pertains to the use of a COMBINATION OF THE INVENTION for the treatment of a proliferative disease and for the preparation of a medicament for the treatment of a proliferative disease.

Moreover, the present invention provides a commercial package comprising as active ingredients COMBINATION OF THE INVENTION, together with instructions for simultaneous, separate or sequential use thereof in the treatment of a proliferative disease.

In one embodiment of the invention, an antidiarrheal agent is administered together with the COMBINATION OF THE INVENTION in order to prevent, control or eliminate diarrhea that is sometimes associated with the administration of epothilones, especially epothilone B. Thus, the present invention also relates to a method of preventing or controlling diarrhea associated with administering an epothilone derivative of formula I, which comprises administering an effective amount of an antidiarrheal agent to the patient receiving treatment with the COMBINATION OF THE INVENTION. Antidiarrheal agents and protocols for their administration are known to those skilled in the art. Antidiarrheal agents suitable for use in the inventive methods and compositions include, but are not limited to, natural opiods, such as tincture of opium, paregoric, and codeine, synthetic opioids, such as diphenoxylate, difenoxin and loperamide, bismuth subsalicylate, octreotide, e.g. as marketed under the trademark SANDOSTATIN™, motilin antagonists and traditional antidiarrheal remedies, such as kaolin, pectin, berberine and muscarinic agents. In particular loperamide or SANDO-STATIN™ can be applied in accordance to the inforamtion provided with the package insert.

## Claims

1. A combination which comprises (a) at least one antineoplastic agent selected from the topoisomerase I inhibitors or topoisomerase II inhibitors epirubicine, doxorubicin, topotecan or 9-nitrocamptothecin, and (b) epothilone B,
in which the active ingredients (a) and (b) are present in each case in free form or in the form of a pharmaceutically acceptable salt and optionally at least on pharmaceutically acceptable carrier; for simultaneous, separate or sequential use.

2. A combination according to claim 1, which is a combined preparation.

3. A pharmaceutical composition comprising a quantity which is jointly therapeutically effective against a proliferative disease of a combination according to claim 1 and at least one pharmaceutically acceptable carrier.

4. A combination according to claim 1 for use in the treatment of a proliferative disease.

5. Use of a combination according to claim 1 for the preparation of a medicament for the treatment of a proliferative

disease.

6. Use according to claim 5, wherein the proliferative disease is a solid tumor disease.

7. A commercial package comprising (a) at least one antineoplastic agent selected from the topoisomerase I inhibitors or topoisomerase II inhibitors epirubicine, doxorubicin, topotecan or 9-nitrocamptothecin, and (b) epothilone B, together with instructions for simultaneous, separate or sequential use thereof in the treatment of a proliferative disease.

## Patentansprüche

1. Kombination, die (a) mindestens ein antineoplastisches Mittel, das aus den Topoisomerase-I-Inhibitoren oder Topoisomerase-II-Inhibitoren Epirubicin, Doxorubicin, Topotecan oder 9-Nitrocamptothecin ausgewählt ist, und (b) Epothilon B, worin die wirksamen Bestandteile (a) und (b) in jedem Fall in freier Form oder in Form eines pharmazeutisch akzeptablen Salzes vorhanden sein können, und optional mindestens einen pharmazeutisch akzeptablen Träger umfasst, zur gleichzeitigen, getrennten oder aufeinanderfolgenden Verwendung.

2. Kombination nach Anspruch 1, die eine kombinierte Zubereitung ist.

3. Pharmazeutische Zusammensetzung, die eine Menge, die gemeinsam therapeutisch gegenüber einer proliferativen Erkrankung wirksam ist, einer Kombination gemäß Anspruch 1 und mindestens einen pharmazeutisch akzeptablen Träger umfasst.

4. Kombination nach Anspruch 1 zur Verwendung bei der Behandlung einer proliferativen Erkrankung.

5. Verwendung einer Kombination gemäß Anspruch 1 zur Herstellung eines Medikaments zur Behandlung einer proliferativen Erkrankung.

6. Verwendung nach Anspruch 5, worin die proliferative Erkrankung eine Erkrankung in Form eines festen Tumors ist.

7. Kommerzielle Packung, die (a) mindestens ein antineoplastisches Mittel, das aus den Topoisomerase-I-Inhibitoren oder Topoisomerase-II-Inhibitoren Epirubicin, Doxorubicin, Topotecan oder 9-Nitrocamptothecin ausgewählt ist, und (b) Epothilon B zusammen mit Instruktionen für eine gleichzeitige, getrennte oder aufeinanderfolgende Verwendung hiervon bei der Behandlung einer proliferativen Erkrankung umfasst.

## Revendications

1. Combinaison qui comprend (a) au moins un agent antinéoplasique choisi parmi les inhibiteurs de la topoisomérase I et les inhibiteurs de la topoisomérase II épirubicine, doxorubicine, topotécan et 9-nitrocamptothécine, et (b) de l'épothilone B, les principes actifs (a) et (b) étant présents dans chaque cas sous forme libre ou sous la forme d'un sel pharmaceutiquement acceptable et, éventuellement, au moins un véhicule pharmaceutiquement acceptable, pour une utilisation simultanée, séparée ou successive.

2. Combinaison selon la revendication 1, qui est une préparation combinée.

3. Composition pharmaceutique comprenant une quantité, qui est conjointement thérapeutiquement efficace contre une maladie proliférative, d'une combinaison selon la revendication 1 et au moins un véhicule pharmaceutiquement acceptable.

4. Combinaison selon la revendication 1 pour utilisation dans le traitement d'une maladie proliférative.

5. Utilisation d'une combinaison selon la revendication 1 pour la préparation d'un médicament destiné au traitement d'une maladie proliférative.

6. Utilisation selon la revendication 5, dans laquelle la maladie proliférative est une maladie à tumeur solide.

7. Produit conditionné destiné à la vente comprenant (a) au moins un agent antinéoplasique choisi parmi les inhibiteurs de la topoisomérase I et les inhibiteurs de la topoisomérase II épirubicine, doxorubicine, topotécan et 9-nitrocamptothécine, et (b) de l'épothilone B, conjointement avec les instructions pour l'utilisation simultanée, séparée ou successive de ceux-ci dans le traitement d'une maladie proliférative.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 9943320 A
- WO 9310121 A
- US 6194181 B
- WO 9825929 A
- WO 9808849 A
- WO 9943653 A
- WO 9822461 A
- WO 0031247 A
- WO 9902514 A
- WO 9967252 A
- WO 0172721 A
- WO 9939694 A
- US 6302838 B

### Non-patent literature cited in the description

- **Bollag et al.** *Cancer Research,* 1995, vol. 55, 2325-33